# EUROPEAN PATENT SPECIFICATION

(11) **EP 3 233 210 B1**
(45) Date of publication and mention of the grant of the patent: **05.02.2020**
(21) Application number: 15817569.5
(22) Date of filing: 14.12.2015
(51) Int. Cl.: A61Q 19/00, C07K 5/09

(54) **TRIPEPTIDES, COMPOSITIONS THEREOF AND THEIR COSMETIC USES**
TRIPEPTIDE, ZUSAMMENSETZUNGEN DAVON UND DEREN KOSMETISCHE VERWENDUNGEN
TRIPEPTIDES, COMPOSITIONS LES CONTENANT ET LEURS UTILISATIONS COSMÉTIQUES

(30) Priority: 16.12.2014 FR 1462511
(43) Date of publication of application: 25.10.2017
(73) Proprietor: Sederma, 78610 Le Perray en Yvelines (FR)
(72) Inventor: PESCHARD, Olivier, 78120 Rambouillet (FR); DOUCET, Anne, 78120 Rambouillet (FR); LEROUX, Richard, 28210 Faverolles (FR); MONDON, Philippe, 92120 Montrouge (FR)
(86) International application number: PCT/IB2015/059581
(87) International publication number: WO 2016/097966

(56) References cited:
- WO-A1-91/12267
- LOREN PICKART ET AL: "GHK and DNA: Resetting the Human Genome to Health", BIOMED RESEARCH INTERNATIONAL, vol. 3, no. 2, 1 January 2014 (2014-01-01) , pages 11-10, XP055220862, US ISSN: 2314-6133, DOI: 10.1517/17425247.2012.735658

### SEQUENCE LISTING

<110> SEDERMA
<120> TRIPEPTIDES, COMPOSITIONS COMPRISING THEM AND USES IN PARTICULAR COSMETIC OF SAID TRIPEPTIDES
<130> KAGEH PCT
<150> FR1462511
   <151> 2014-12-16
<160> 19
<170> PatentIn version 3.5
<210> 1
   <211> 5
   <212> PRT
   <213> artificial sequence
<220>
   <223> synthetic peptide
<220>
   <221> MOD_RES
   <222> (1)..(1)
   <223> amidation by a Palmitoyl chain
<400> 1
   Lys Thr Thr Lys Ser
   1               5
<210> 2
   <211> 4
   <212> PRT
   <213> artificial sequence
<220>
   <223> synthetic peptide
<220>
   <221> MOD_RES
   <222> (1)..(1)
   <223> amidation by a Palmitoyl chain
<400> 2
   Gly Gln Pro Arg
   1
<210> 3
   <211> 6
   <212> PRT
   <213> artificial sequence
<220>
   <223> synthetic peptide
<220>
   <221> MOD_RES
   <222> (1)..(1)
   <223> amidation by a Palmitoyl chain
<400> 3
   Val Gly Val Ala Pro Gly
   1               5
<210> 4
   <211> 4
   <212> PRT
   <213> artificial sequence
<220>
   <223> synthetic peptide
<400> 4
   Arg Ser Arg Lys
   1
<210> 5
   <211> 4
   <212> PRT
   <213> artificial sequence
<220>
   <223> synthetic peptide
<400> 5
   Gly Gln Pro Arg
   1
<210> 6
   <211> 4
   <212> PRT
   <213> artificial sequence
<220>
   <223> synthetic peptide
<400> 6
   Lys Thr Phe Lys
   1
<210> 7
   <211> 5
   <212> PRT
   <213> artificial sequence
<220>
   <223> synthetic peptide
<400> 7
   Lys Thr Thr Lys Ser
   1               5
<210> 8
   <211> 6
   <212> PRT
   <213> artificial sequence
<220>
   <223> synthetic peptide
<400> 8
   Gly Lys Thr Thr Lys Ser
   1               5
<210> 9
   <211> 6
   <212> PRT
   <213> artificial sequence
<220>
   <223> synthetic peptide
<400> 9
   Val Gly Val Ala Pro Gly
   1               5
<210> 10
   <211> 5
   <212> PRT
   <213> artificial sequence
<220>
   <223> synthetic peptide
<220>
   <221> MOD_RES
   <222> (1)..(1)
   <223> Amidation by a Palmitoyl chain
<400> 10
   Tyr Gly Gly Phe Leu
   1               5
<210> 11
   <211> 5
   <212> PRT
   <213> artificial sequence
<220>
   <223> synthetic peptide
<220>
   <221> MOD_RES
   <222> (1)..(1)
   <223> Amidation by a palmitoyl chain
<400> 11
   Tyr Gly Gly Phe Pro
   1               5
<210> 12
   <211> 6
   <212> PRT
   <213> artificial sequence
<220>
   <223> synthetic peptide
<400> 12
   His Leu Asp Ile Ile Trp
   1               5
<210> 13
   <211> 6
   <212> PRT
   <213> artificial sequence
<220>
   <223> Synthetic peptide
<220>
   <221> MISC_FEATURE
   <222> (6)..(6)
   <223> Xaa is aTryptoline-3-carboxylic acid residu (Tpi)
<400> 13
   His Leu Asp Ile Ile Xaa
   1               5
<210> 14
   <211> 6
   <212> PRT
   <213> artificial sequence
<220>
   <223> Synthetic peptide
<400> 14
   His Leu Asp Ile Ile Phe
   1               5
<210> 15
   <211> 6
   <212> PRT
   <213> artificial sequence
<220>
   <223> synthetic peptide
<220>
   <221> MOD_RES
   <222> (1)..(1)
   <223> Acetylation
<220>
   <221> MOD_RES
   <222> (6)..(6)
   <223> NH2
<400> 15
   Glu Glu Met Gln Arg Arg
   1               5
<210> 16
   <211> 5
   <212> PRT
   <213> artificial sequence
<220>
   <223> synthetic peptide
<400> 16
   Tyr Ala Gly Phe Leu
   1               5
<210> 17
   <211> 4
   <212> PRT
   <213> artificial sequence
<220>
   <223> synthetic peptide
<220>
   <221> MOD_RES
   <222> (1)..(1)
   <223> Acetylation
<400> 17
   Ala His Ser His
   1
<210> 18
   <211> 6
   <212> PRT
   <213> artificial sequence
<220>
   <223> synthetic peptide
<400> 18
   Ser Ile Lys Val Ala Val
   1               5
<210> 19
   <211> 6
   <212> PRT
   <213> artificial sequence
<220>
   <223> synthetic peptide
<400> 19
   Gly Pro Gln Gly Pro Gln
   1               5

## Description

### TECHNICAL FIELD

The present invention relates to tripeptides, compositions comprising them, and cosmetic uses of said peptides. It relates more particularly to tripeptides for treating the skin and appendages of mammals, humans or animals.

The cosmetics (topically or orally applications), hygiene and personal care and dermo-pharmacy industries are concerned.

### BACKGROUND ART

Peptides have a significant signal function and coordinate many biochemical processes. Thus they are essential and promising active ingredients especially in the cosmetics industry where new compounds are always sought, that are capable of beautifying skin and/or its appendages, that is to say to improve their general condition.

The present inventors are more particularly interested in the research of novel peptides having an activity on the main molecules constituents of the skin extracellular matrix (ECM) which quantity and quality decrease with aging (chronological or premature), and more particularly peptides active on the synthesis of type I collagen and elastin and also active on the synthesis of glycoproteins like fibronectin.

Loss of density and thickness of dermis are closely related to a reduced synthesis of the macromolecules with aging by fibroblasts, the cells responsible for their production. Collagen I is the most abundant protein in the dermis. It is essential in obtaining a firm skin. Elastin is synthesized and secreted in the dermal extracellular space. It is the major component up to 90% of elastic fibers. Fibronectin is a glycoprotein also present in the extracellular matrix and which plays a key role in cell adhesion to the extracellular matrix. It can simultaneously bind to the cell and to other molecules of the extracellular matrix, such as collagen or another fibronectin molecule. To this aim, the fibronectin molecules assemble together to form adhesive elastic fibers on the surface of many cells. This determines the mechanical properties (elasticity, flexibility and firmness) of the skin.

The increase in collagen IV and laminins is also sought. It helps to restore/enhance the dermal/epidermal junction (DEJ). Collagen IV forms a two-dimensional network and constitutes a major component of the dermal/epidermal junction. Laminins are also contained in the basal layer and are involved in anchoring cell surfaces to basal lamina. These two essential components ensure together that keratinocytes of the basal lamina have a better anchoring and contribute to maintaining the flexibility of the skin.

The reduction in protein synthesis with aging is perceived at the DEJ level. Type IV collagen is more fragmented and at the same time less produced, as well as laminins, resulting in some areas an impaired DEJ and poorer communication between melanocytes, keratinocytes and DEJ, and less flexibility of the system. Therefore, the interest to stimulate the synthesis of these two proteins appears clearly.

The stimulated synthesis of these molecules will generate results on the beautifying and general condition of the skin, at the level of mechanical properties: a skin that will be denser, thicker, replumped, firmer, more toned, more supple and elastic, the peptide having a volumizing, plumping effect and thus anti-wrinkles, and at the level of complexion imperfections (more homogeneous color and more brightness).

Many peptides or peptide mixtures having properties on the ECM and anti-aging applications have already been proposed, including by the Applicant, as the Pal-KTTKS (SEQ ID NO: 1) sold under the MATRIXYL™ trademark, the Pal-GHK and Pal-GQPR (SEQ ID NO: 2) mixture sold under the MATRIXYL 3000™ trademark or more recently the Pal-KMO₂K sold under the trademark MATRIXYL Synthe'6™ (MO₂ corresponding to a dioxygenated methionine). Other known peptides are mentioned below in the specification.

The use of the tripeptide GHK for treating skin is disclosed in the scientific publication "GHK and DNA: Resetting the Human Genome to Health", Loren Pickart et al., Biomed Research International, vol.3, no.2, 1 january 2014 (2014-01-01), pages 11-10.

The present invention aims to propose other peptides capable of improving the general condition of the skin and its appendages, and particularly peptides active on the synthesis of ECM proteins. It also aims to provide sufficiently effective peptides to be used alone or in combination, in proportions of a few ppm, and that can be used as a topical composition, in particular a cosmetic composition.

### SUMMARY OF THE INVENTION

The present invention provides a tripeptide according to claim 1.

Thus, the peptides of the invention are modified at their N-terminal end and/or their C-terminal end, preferably at their N-terminal end or their C-terminal end, more preferably their N-terminal end.

The detailed description of *in vitro* tests given below shows that these peptides exhibit activity on the molecule markers of the ECM, active from a few ppm, and may be used individually or in combination to improve the appearance and the general state of the skin and its appendages, and in particular for the treatment and/or prevention of signs of aging and/or imperfections of skin and its appendages. The inventors have shown that peptides according to the invention exhibited in particular a pro-collagen activity, including regarding collagen 1 and collagen 4 and a pro-elastin activity.

According to other preferred features of the invention:
- R₁ and/or R₂ is a lipophilic alkyle chain of 3 to 24 carbon atoms; and/or
- X is an acyle radical -CO-R₁ and Z is selected from OH, OMe, OEt and NH₂, preferably OH; X is preferably selected from an octanoyl (C₈), decanoyl (C₁₀), lauroyl (C₁₂), myristoyl (C₁₄), palmitoyl (C₁₆), stearoyl (C₁₈), biotinoyl, elaidoyl, oleoyl and lipoyl; more preferably selected from a lauroyl (C₁₂), myristoyl (C₁₄) and palmitoyl (C₁₆); and/or
- Z is OH and X is selected from a palmitoyl (C₁₆), myristoyl (C₁₄) and lauroyl (C₁₂); more preferably a palmitoyl (C₁₆).

The preferred peptides of the invention are the Pal-KGH-OH and the Pal-KHG-OH.

The peptides of the invention may be optically pure, or be composed of L or D isomers or a mixture thereof. L isomers, which are those found in nature, may be preferred.

The peptides may be in the form of salts, especially salts of hydrochloric acid or acetic acid, or any salts commonly used in cosmetics.

Derivatives (with modification and/or addition of a chemical function but without change in the carbon skeleton) and analogs (with modification and/or addition of a chemical functional group but with an additional change in the carbon skeleton), complexes with other species such as metal ion (eg copper, zinc, manganese, magnesium, and others) are also disclosed.

The present invention also provides a composition, especially topical, comprising at least one peptide according to the invention in a physiologically acceptable medium. According to the excipient and the peptide dosage, this composition will constitute for example a concentrated active ingredient or a final composition less concentrated directly for the client or patient.

"Physiologically acceptable medium" means according to the present invention, without limitation, an aqueous or hydro-alcoholic solution, a water-in-oil emulsion, an oil-in-water emulsion, a microemulsion, an aqueous gel, an anhydrous gel, a serum, a dispersion of vesicles, or a powder.

"Physiologically acceptable" means that the compositions are suitable for topical or transdermal use, in contact with mucous membranes, appendages (nails, hairs), scalp and skin of mammals, particularly human, compositions which may be ingested, or injected into the skin, without risk of toxicity, incompatibility, instability, allergic response, and others.

This "physiologically acceptable medium" forms what is commonly called the excipient of the composition.

The peptides of the invention can be solubilized in a hydrophilic or lipophilic matrix with optionally a solubilizer, according to the envisaged use.

The peptide may be combined with other active ingredients at effective concentrations that can act synergistically or additionnaly for reinforcing and achieveing the desired effects described for the invention, such as the following agents: anti-aging, anti-fine lines and wrinkles, lightening, propigmenting, hydrating, moisturizing, humectant, slimming, exfoliating, anti-acne, anti-redness, anti-inflammatories, anti-oxidant/radical scavengers, acting on brightness of complexion, anti-glycation, volumizing, restructuring, anti-carbonylation, dermo-relaxing, anti-hair regrowth, action on stratum corneum, dermal-epidermal junction, HSP protein production, firmness, elasticity and tone of skin, hair growth (eyelashes and eyebrows), eye contours (dark circles and under eye bags), promoting blood circulation, other peptides, vitamins etc. These active ingredients may be obtained from plant materials, such as classical plant extracts or products of plant cell culture or fermentation.

More specifically, the peptides according to the invention may be combined with at least one of compounds selected from compounds of the vitamin B3, compounds such as niacinamide or tocopherol, retinoid compounds such as retinol, hexamidine, α-lipoic acid, resveratrol or DHEA, hyaluronic acid, peptides, in particular the N-acetyl-Tyr-Arg-O-hexadecyl ester, Pal-VGVAPG (SEQ ID NO: 3), Pal-KTTKS (SEQ ID NO: 1), Pal-GHK, Pal-KMO2K and Pal-GQPR (SEQ ID NO: 2), which are widely used active ingredients in topical cosmetic or dermopharmaceutical compositions.

The composition according to the invention may be applied to the face, body, neckline, scalp, hair, eyelashes, body hair, in whatever form or carriers known to those skilled in the art, in particular in the form of solution, dispersion, emulsion, paste, or powder, individually or as a premix or in vehicles individually or as a premix in vectors such as macro-, micro-, or nano-capsules, macro-, micro- or , nano-spheres, liposomes, oleosomes or chylomicrons, macro-, micro-, or nanoparticles or macro-, micro- or nano-sponges, micro- or nano-emulsions or adsorbed on organic polymer powders, talcs, bentonites, spores or exines, and other inorganic or organic supports.

In cosmetics, applications can be offered particularly in skincare ranges for the face, body, hair and body hairs, and in make-up ranges, including for eyebrows and eyelashes.

In general, the peptides according to the present invention may be used in any form, in a form bound to or incorporated in or absorbed in or adsorbed on macro-, micro-, and nanoparticles, or macro-, micro-, and nano-capsules, for the treatment of textiles, natural or synthetic fibers, wools, and any materials that may be used for clothing or underwear for day or night intended to come into contact with the skin, handkerchiefs or cloths, to exert their cosmetic effect via this skin/textile contact and to permit continuous topical delivery.

The CTFA (« International Cosmetic Ingredient Dictionary & Handbook » (15th Ed. 2014) published by « the Personal Care Products council », ex- « the Cosmetic, Toiletry, and Fragrance Association, Inc. », Washington, D.C.), describes a non-limited wide variety of cosmetic and pharmaceutical ingredients commonly used in the skin care industry, which are suitable for use as additional ingredients in the compositions according to the present invention.

Further additional skin care actives that are particularly useful can be found in the commercial literature of Sederma and on the website www.sederma.com.

The following commercial actives can also be mentioned, as examples: betaine, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Argireline™ (commercial name for the acetyl hexapeptide-3 of Lipotec), spilanthol or an extract of *Acmella oleracea* known under the commercial name Gatuline Expression™, an extract of *Boswellia serrata* known under the commercial name Boswellin™, Deepaline PVB™ (Seppic), Syn-AKE™ (Pentapharm), Ameliox™, Bioxilift™ (Silab), PhytoCellTec™Argan (Mibelle), Papilactyl D™ (Silab), Preventhelia™ (Lipotec), and from Sederma: Subliskin™, Venuceane™, Moist 24™, Vegesome Moist 24™, Essenskin™, Juvinity™, Revidrat™, Resistem™, Chronodyn™, Kombuchka™, Chromocare™, Calmosensine™, Glycokin factor S™, Biobustyl™, Idealift™, Ceramide 2™, Ceramide A2™ et Ceramide HO3™, Legance™, Intenslim™, Prodizia™, Beautifeye™, NG-shea butter unsaponifiables (natural grade), Zingerslim™, Meiritage™, Senestem™, Sebuless™, Majestem™, Apiscalp™, Rubistem™, or mixture thereof.

Among other plant extracts which can be combined with the peptide of the invention, there may more particularly be mentioned extracts of Ivy, in particular English Ivy *(Hedera Helix),* of *Bupleurum chinensis,* of *Bupleurum Falcatum,* of arnica (*Arnica Montana L*)*,* of rosemary (*Rosmarinus officinalis N*)*,* of marigold (*Calendula officinalis*)*,* of sage (*Salvia officinalis L*)*,* of ginseng (*Panax ginseng*)*,* of ginko biloba, of St.-John's-Wort (*Hyperycum Perforatum*), of butcher's-broom (*Ruscus aculeatus L*)*,* of European meadowsweet (*Filipendula ulmaria L*)*,* of big-flowered Jarva tea (*Orthosiphon Stamincus Benth*)*,* of algae (*Fucus Vesiculosus*)*,* of birch (*Betula alba*)*,* of green tea, of cola nuts (*Cola Nipida*), of horse-chestnut, of bamboo, of *Centella asiatica,* of heather, of fucus, of willow, of mouse-ear, of escine, of cangzhu, of *chrysanthellum indicum,* of the plants of the *Armeniacea* genus, *Atractylodis Platicodon, Sinnomenum, Pharbitidis, Flemingia,* of *Coleus* such as *C*. *Forskohlii, C. blumei, C. esquirolii, C. scutellaroides, C. xanthantus* and *C*. *Barbatus,* such as the extract of root of *Coleus barbatus,* extracts of *Ballote,* of *Guioa,* of *Davallia,* of *Terminalia,* of *Barringtonia,* of *Trema,* of *antirobia, cecropia, argania, dioscoreae* such as *Dioscorea opposita* or Mexican, extracts of *Ammi visnaga,* of *Siegesbeckia,* in particular *Siegesbeckia orientalis,* vegetable extracts of the family of *Ericaceae,* in particular bilberry extracts (*Vaccinium angustifollium*) or *Arctostaphylos uva ursi, aloe vera,* plant containing sterols (e.g., phytosterol), Manjistha (extracted from plants of the genus *Rubia,* particularly *Rubia Cordifolia*)*,* and Guggal (extracted from plants of the genus *Commiphora,* particularly *Commiphora Mukul*)*,* kola extract, chamomile, red clover extract, Piper methysticum extract (Kava Kava™ from Sederma), *Bacopa monieri* extract (Bacocalmine™ from Sederma) and sea whip extract, extracts of *Glycyrrhiza glabra,* of mulberry, of *melaleuca* (tea tree), of *Larrea divaricata,* of *Rabdosia rubescens,* of *Euglena gracilis,* of *Fibraurea recisa Hirudinea,* of *Chaparral Sorghum,* of sun flower extract, of *Enantia chlorantha,* of Mitracarpe of *Spermacocea* genus, of *Buchu barosma,* of *Lawsonia inermis L.,* of *Adiantium Capillus-Veneris L.,* of *Chelidonium majus,* of *Luffa cylindrica,* of Japanese Mandarin (*Citrus reticulata Blanco* var. *unshiu*)*,* of *Camelia sinensis,* of *Imperata cylindrica,* of *Glaucium Flavum,* of *Cupressus Sempervirens,* of *Polygonatum multiflorum,* of *loveyly hemsleya,* of *Sambucus Nigra,* of *Phaseolus lunatus,* of *Centaurium,* of *Macrocystis Pyrifera,* of *Turnera Diffusa,* of *Anemarrhena asphodeloides,* of *Portulaca pilosa,* of *Humulus lupulus,* of *Coffea Arabica,* of *Ilex Paraguariensis,* or of *Globularia Cordifolia,* of *Albizzia julibrissin,* of *Oxydendron arboretum,* of *Zingimber Zerumbet Smith,* of *Astragalus membranaceus,* of *Atractylodes macrocephalae,* of *Plantago lanceolata,* of *Leontopodium alpinum,* of *Mirabilis jalapa* or of *Apium graveolens.*

The compositions of the present invention may include other peptides, including, without limitation, di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5% by weight. According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides and which are found in nature, and/or are commercially available.

Suitable dipeptides for use herein include but are not limited to Carnosine (beta-AH), YR, VW, NF, DF, KT, KC, CK, KP, KK or TT. Suitable tripeptides for use herein include, but are not limited to RKR, HGG, GHK, GKH, GGH, GHG, KFK, GKH, KPK, KMOK, KMO2K or KAvaK. Suitable tetrapeptides for use herein include but are not limited to RSRK (SEQ ID NO: 4), GQPR (SEQ ID NO: 5) or KTFK (SEQ ID NO: 6). Suitable pentapeptides include, but are not limited to KTTKS (SEQ ID NO: 7). Suitable hexapeptides include but are not limited to GKTTKS (SEQ ID NO: 8) and VGVAPG (SEQ ID NO: 9).

Other suitable peptides for use herein include, but are not limited to: lipophilic derivatives of peptides, preferably palmitoyl derivatives, and metal complexes as aforementioned (e.g. copper complex of the tripeptide HGG). Preferred dipeptide include for example N-Palmitoyl-beta-Ala-His, N-Acetyl-Tyr-Arg-hexadecylester (Calmosensine™, Idealift™ from Sederma). Preferred tripeptide derivatives include for example N-Palmitoyl-Gly-Lys-His, and Pal-Gly-His-Lys, (Pal-GKH and Pal-GHK from Sederma), the copper derivative of HGG (Lamin™ from Sigma), Lipospondin (N-Elaidoyl-KFK) and its analogs of conservative substitution, N-Acetyl-RKR-NH₂ (Peptide CK+), N-Biot-GHK (from Sederma), Pal-KMO2K (Matrixyl Synthe6™ from Sederma) and derivatives thereof. Suitable tetrapeptide derivatives for use according to the present invention include, but are not limited to, N-Pal-GQPR (SEQ ID NO: 2) (from Sederma), suitable pentapeptide derivatives for use herein include, but are not limited to, Pal-KTTKS (SEQ ID NO: 1) (available as Matrixyl™ from Sederma), Pal-YGGFL (SEQ ID NO: 10) or Pal-YGGFP (SEQ ID NO: 11) or mixtures thereof. Suitable hexapeptide derivatives for use herein include, but are not limited to, Pal-VGVAPG (SEQ ID NO: 3), HLDIIW (SEQ ID NO: 12), HKDIITpi (SEQ ID NO: 13), Tpi being the Tryptoline-3-carboxylic acid residue, or HLDIIF (SEQ ID NO: 14), or Pal-, and derivatives thereof. The mixture of Pal-GHK and Pal-GQPR (SEQ ID NO: 2) (Matrixyl™ 3000, Sederma) can also be mentioned.

The preferred compositions commercially available containing a tripeptide or a derivative include Biopeptide-CL™, Maxilip™, Biobustyl™, Procapil™ and Matrixyl™synthe'6™ of Sederma. The compositions commercially available preferred sources of tetrapeptides include Rigin™, Eyeliss™, Matrixyl™ Reloaded and Matrixyl 3000™ which contain between 50 and 500 ppm of Pal-GQPR (SEQ ID NO: 2) and an excipient, proposed by Sederma.

The following marketed peptides can be mentioned as well as additional active ingredients:
- Vialox™ (INCI name = Pentapeptide-3 (synthetic peptide comprising alanine, arginine, isoleucine, glycine and proline)), Syn-ake™ (β-Ala-Pro-Dab-NH-Bzl) or Syn-Coll™ (Pal-Lys-Val-Lys-OH) marketed by Pentapharm;
- Argireline™ (Ac-Glu-Glu-Met-Gln-Arg-Arg-NH₂ (INCI name = Acetyl hexapeptide-3) (SEQ ID NO: 15), Leuphasyl™ (Tyr-D-Ala-Gly-Phe-Leu) (SEQ ID NO: 16), Aldenine™ (Gly-His-Lys), Trylagen™ (INCI name = Pseudoalteromonas Ferment Extract, Hydro lyzed Wheat Protein, Hydro lyzed Soy Protein, Tripeptide-10 Citrulline (reaction product of Citrulline and Tripeptide-10 (synthetic peptide constituted of aspartic acid, isoleucine and lysine)), Tripeptide-1), Eyeseryl™ (Ac-β-Ala-His-Ser-His)(SEQ ID NO: 17), Serilesine™ (Ser-Ile-Lys-Val-Ala-Val) (SEQ ID N°18) or Decorinyl™ (INCI name: Tripeptide-10 Citrulline = reaction product of Citrulline and Tripeptide-10 (synthetic peptide constituted of aspartic acid, isoleucine and lysine) marketed by Lipotec;
- Collaxyl™ (Gly-Pro-Gln-Gly-Pro-Gln (SEQ ID N°19)) or Quintescine™ (Cys-Gly) marketed by Vincience;
- Cytokinol™LS (casein hydrolysate) marketed by Les Laboratoires Serobiologiques/Cognis;
- Kollaren™ (Gly-His-Lys), IP2000™ (Pal-Val-Tyr-Val) or Meliprene™ (INCI name = Monofluoroheptapeptide-1: reaction product of acetic acide and a synthetic peptide comprising arginine, glycine, glutamic acid, histidine, norleucine, p-fluorophenylalanine and tryptophan) marketed by l'Institut Européen de Biologie Cellulaire;
- Neutrazen™ (Pal-His-D-Phe-Arg-NH₂) marketed by Innovations; or
- BONT-L-Peptide™ (INCI name = Palmitoyl Hexapeptide-19: reaction product of palmitic acid and Hexapeptide-19 (synthetic peptide constituted of asparagine, aspartic acid, lysine and methionine), Timp-Peptide™ (INCI name = Acetyl Hexapeptide-20: reaction product obtained by acetylation of Hexapeptide-20 (synthetic peptide constituted of alanine, glycine, lysine, valine and proline) or ECM Moduline™ (INCI name = Palmitoyl Tripeptide-28: reaction product of palmitic acid and Tripeptide-28 (synthetic peptide constituted of arginine, lysine and phenylalanine) marketed by lnfinitec Activos.

The present invention thus provides the use of at least one peptide of the invention or a composition comprising the latter, as defined above, for a non-therapeutic cosmetic treatment to improve the general condition of skin and/or its appendages and to treat the imperfections.

Preferably according to the invention, the treatment is topical.

The peptide of the invention is more particularly preconized according to the invention for an anti-aging treament, in particular:
- A treatment of wrinkles and fine lines; and/or
- A treatment for improving the mechanical properties of the skin: firmness, tone, elasticity and/or flexibility; and/or
- A treatment for increasing density and volume of skin (volumizing, plumping and/or restructuring effect); and/or
- A treatment for fighting strechmarks, and/or
- A treatment for improving homogenity and radiance (brightness) of complexion.

Other applications are of course conceivable for the tripeptides of the invention (alone or combined), for example moisturizing, slimming, detoxifying, anti-glycation, anti-radical, tensor, anti-fatigue, anti-under eye bags and/or dark circles , calming, effect on hair growth, action on pigmentation, scalp, etc. as a preventative or curative measure.

The present invention encompasses a method of topical cosmetic treatment, non-therapeutical, for improving the appearance and general condition of the skin and its appendages, comprising the topical application to the skin of a subject in need thereof of an effective amount of a peptide or mixture of peptides of the invention or a composition according to the invention comprising the said peptide or mixture of peptides, the peptides being as defined above.

« Topical treatment » or "topical use" means according to the invention, an application that is intended to act where it is applied: skin, mucosa and/or appendages.

The peptide or composition of the invention may be applied locally to targeted areas.

The "effective" amount of the active peptide or mixture of peptides in the composition, that is to say its dosage, depends on various factors, such as the age, the condition of the skin and appendages of the person, seriousness of the disorder(s), the administration mode, etc. An effective amount means a non-toxic amount enough to achieve the desired effect.

In a cosmetic composition according to the invention, the peptide(s) to be present in an effective amount, is generally present in an amount ranging from 0.000001% and 15% with regard to the total weight of the composition, preferably from 0.00001% to 5%, more preferably from 0.0001% to 0.01% (from 1 to 100 ppm) for a topical cosmetic application, depending on the destination of the composition and the desired effect more or less pronounced. The peptides may be present in the compositions according to the invention in varying relative proportions, in equivalent amounts, or on the contrary in different proportions.

All percentages and ratios used herein are by weight of the total composition and all measurements are made at 25°C unless it is otherwise specified.

For example, for a cosmetic treatment of the face, the European Cosmetics Directive has set a standard amount for applying a cream of 2.72mg/cm²/day/person and for a body lotion of 0.5mg/cm²/day/person.

According to other specific features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising one peptide of the invention, and in a second compartment a composition containing another active ingredient and/or excipient, the compositions contained in the said first and second compartments in this case being considered to be a combination composition for simultaneous, separate or stepwise use in time, particularly in one of the treatment methods recited above.

The treatment according to the invention is more particularly adapted to slow down the degradation of the molecules of the dermal extracellular matrix and/or to act on the DEJ via the stimulation of collagen IV and/or laminin.

Other uses are of course possible for the peptides of the invention (alone or in combination), for example moisturizing, slimming, detoxifying, anti-glycation, anti-radical, toning, tired skins, under-eye bags and/or dark circles, calming, hair growth, radiance of complexion, pigmentation, scalp treatments, etc., for either prevention or cure.

### DETAILED DESCRIPTION

The following examples disclose and illustrate certain aspects of the invention. They should not be seen as limiting the disclosure, as they only provide useful information for its understanding and implementation.

### A) Example of synthesis of a peptide of the invention: the Pal-KGH-OH

The Pal-KGH-OH peptide is prepared by peptidic synthesis. Histidine is coupled with a resin *via* its terminal acid function (with a coupling agent, e.g. DCC (diclyclohexylcarbodiimide)/NHS (N-hydroxysuccinimide) or HBTU (2-(1H-benzotriazole-1-yl)-1, 1, 3, 3-tetramethyluronium hexafluorophosphate) / HOBT (1-hydroxy-benzotriazole)). Then the histidine thus protected is reacted with a glycine derivative in the presence of a coupling agent, and then the same operation is carried out to add lysine. The later is then acylated on its amine function with an activated derivative of palmitic acid (palmitoyl chloride for example) in the presence of a base. After precipitation, washing and drying, the palmitoyl-lysyl-glycyl-histidine product is obtained in solid form.

This same method of synthesis can be applied to the peptides of formula I according to the invention, for example to the Pal-KHG-OH peptide.

### B) Preparing a composition according to the invention comprising the Pal-KGH-OH peptide of example A

### Starting products:

- The pure peptide, synthetized according to the synthesis method explained above;
- Excipient: a mixture of fatty esters, chosen in order to form an oily matrix, for example for forming a water free composition for the further formulation of water free cosmetic compositions.

*Operating mode:* The peptide is mixed with the excipient and put under gentle stirring and heating until solubilization and total clarity.

### C) In vitro evaluations

The peptides according to the invention show a number of remarkable effects presented below. Peptides prepared according to A) above and dissolved in an excipient were *in vitro* tested and showed activities that are presented below.

### 1) ELISA assays

### Protocol

Normal human fibroblasts (NHF) in culture are brought into contact with the products to be tested or their excipient (negative control) for 72 hours. After the contact, the culture supernatants are recovered and the synthesis of dermal macromolecules are estimated by ELISA. An estimation of cell viability is performed by Hoechst assay and is used to weight the obtained data.

### Results

**Table 1: Collagen I**

| **Product** | **Concentration** | **Change % / control** | **Significance (Student test)** |
|---|---|---|---|
| **Pal-KGH-OH** | 3 ppm | +69 | *p*< *0.05* |
| | 7 ppm | +123 | *p<0.01* |
| **Pal-KHG-OH** | 3 ppm | +66 | *p<0.05* |
| | 7 ppm | +116 | *p<0.01* |
| | 12.5 ppm | + 132 | *p<0.01* |

**Table 2: Collagen IV**

| **Product** | **Concentration** | **Change % / control** | **Significance (Student test)** |
|---|---|---|---|
| **Pal-KGH-OH** | 7 ppm | + 35 | *p< 0.01* |
| | 10 ppm | + 74 | *p<0.01* |
| | 12.5 ppm | + 199 | *p<0.01* |
| **Pal-KHG-OH** | 7 ppm | + 62 | *p<0.01* |
| | 10 ppm | + 109 | *p<0.01* |
| | 12.5 ppm | + 183 | *p<0.01* |

**Table 3: Fibronectin**

| **Product** | **Concentration** | **Change % / control** | **Significance (Student test)** |
|---|---|---|---|
| **Pal-KGH-OH** | 7 ppm | + 40 | *p<0.05* |
| | 10 ppm | + 45 | *p<0.05* |
| | 12.5 ppm | + 79 | *p<0.01* |
| **Pal-KHG-OH** | 7 ppm | + 46 | *p<0.01* |
| | 10 ppm | + 50 | *p<0.01* |
| | 12.5 ppm | + 91 | *p<0.01* |

**Table 4: Elastin**

| **Product** | **Concentration** | **Change % / control** | **Significance (Student test)** |
|---|---|---|---|
| **Pal-KHG-OH** | 3 ppm | +139 | *p< 0.05* |
| | 7 ppm | +113 | *p<0.01* |

The results show that the peptides according to the invention stimulate the synthesis of collagen I and IV, fibronectin, laminin and elastin for the Pal-KHG on normal human fibroblasts at concentrations of a few ppm and in significant proportions.

### 2) Immunofluorescence Assays

### Protocol

Normal human fibroblasts (NHF) are cultured for 24h. The cells are contacted or not with the products to be tested or their excipient at various concentrations for 6 days for collagen I or 14 days for elastin (DMEMc 5% FCS). The synthesis of type I collagen and elastin produced by the cells in the form of extracellular matrix is then quantified by immuno-marking on the fixed layers. A counting of the nuclei Hoechst labeled is carried out in parallel in order to have an estimate of viability and to weight the data.

### Results

**Table 5: Collagen I**

| **Product** | **Concentration** | **Change % / control** | **Significance (Student test)** |
|---|---|---|---|
| **Pal-KGH-OH** | 3 ppm | + 408.8 | *p< 0.01* |
| | 7 ppm | + 724.4 | *p<0.01* |
| | | + 399.4 | *p<0.01* |
| **Pal-KHG-OH** | 3 ppm | + 133.8 | *p<0.01* |
| | 10 ppm | + 644.8 | *p<0.01* |
| | 15 ppm | + 394.9 | *p<0.01* |

**Table 6: Elastin**

| **Product** | **Concentration** | **Change % / control** | **Significance (Student test)** |
|---|---|---|---|
| **Pal-KHG-OH** | 3 ppm | + 59.1 | *p< 0.01* |
| | 7 ppm | + 41.2 | *p<0.01* |
| | 15 ppm | + 51.5 | *p<0.01* |
| **Pal-KGH-OH** | 3 ppm | +82.8 | *p<0.01* |
| | 7 ppm | + 76.3 | *p<0.01* |
| | | + 126.8 | *p<0.01* |

**Table 7: Collagen IV**

| **Product** | **Concentration** | **Change % / control** | **Significance (Student test)** |
|---|---|---|---|
| **Pal-KHG-OH** | 3 ppm | + 215.2 | *p< 0.01* |
| | 7 ppm | + 504.9 | *p<0.01* |
| | 15 ppm | + 436.8 | *p<0.01* |
| **Pal-KGH-OH** | 3 ppm | +323.7 | *p<0.01* |
| | 7 ppm | +274.4 | *p<0.01* |
| | | +259.5 | *p<0.01* |

**Table 8: Hyaluronic acid**

| **Product** | **Concentration** | **Change % / control** | **Significance (Student test)** |
|---|---|---|---|
| **Pal-KGH-OH** | 3 ppm | +37.6 | *p<0.01* |
| | 7 ppm | +42.2 | *p<0.05* |

### D) GALENIC

Different formulations are described below. Additional cosmetic active ingredients, brought optionally in support and/or in addition to the activity of the active ingredient according to the invention can be added in the correct phase according to their hydrophobic or hydrophilic nature. These ingredients can be of any category according to their(s) function(s), site of application (body, face, neck, chest, hands, hair, eyelashes, eyebrows, hair, etc.), the desired final effect and the targeted consumer, for example antioxidant, moisturizing, nourishing, protective, smoothing, remodeling, volumizing (lipofiling), acting on skin radiance, anti-spots, anti-dark circles, anti-glycation, slimming, relaxing, myorelaxant, anti-redness, anti-stretch marks, etc. They are mentioned above in the description.

**1) Cream form, for example an antiaging day cream for the face**

| **Ingredient (INCI name)** | **Weight %** |
|---|---|
| **Phase A** | |
| Sorbitan Stearate | 3.00 |
| Cyclopentasiloxane (and) Cyclohexasiloxane | 2.00 |
| Ethylhexyl Palmitate | 3.00 |
| Glyceryl Stearate (and) PEG-100 Stearate | 3.00 |
| Ethylhexyl Methoxycinnamate | 1.00 |
| Ethylhexyl Dimethyl PABA | 1.00 |

| **Phase B** | |
|---|---|
| Demineralised water | Qsp 100 |
| Ultrez 10 (Carbomer) | 0.40 |

| **Phase C** | |
|---|---|
| Glycerin | 5.00 |
| Preservative | qs |

| **Phase D** | |
|---|---|
| **Peptide according to the invention in a fatty excipient** | 3.00 |

| **Phase E** | |
|---|---|
| Potassium Sorbate | 0.10 |

| **Phase F** | |
|---|---|
| Sodium Hydroxide 30% | 0.60 |
| Demineralised water | 6.00 |

| **Phase G** | |
|---|---|
| Fragrance | 0.10 |

Protocol: Weigh phase A and heat at 75°C in a water bath. Weigh phase B and let swell for 20 minutes. Melt phase C until dissolved and add to phase B. Heat phase (B+C) at 75°C using a water bath. Pour phase A into phase (B+C) under Staro stirring. Extemporaneously, add phase D to phase (A+B+C). At approximately 45°C add phase E and neutralize with phase F. Mix well. At 35°C, add phase G. Homogenize. pH: 6.20.

### Examples of ingredients which may be added to this formulation:

- CALMOSENSINE™: soothing active for sensitive skins marketed by Sederma (WO1998/07744) comprising the Tyr-Arg lipo-dipeptide. It reduces discomfort feelings.
- SEBULESS™: purifying sebo-regulator ingredient comprising a *Syringa vulgaris* extract, marketed by Sederma, which mattifies and refreshes complexion, fades the blemishes.
- PRODIZIA™: active ingredient marketed by SEDERMA (WO2013/046137), comprising an extract of *Albizia julibrissin,* fighting the signs cutaneous fatigue: dark circles, under eye bags, dull complexion and drawn features, by repairing and protection the skin against the caused by damages of glycation and glycoxydation.
- PACIFEEL™: active ingredient actif marketed by Sederma, comprising a natural extract of the *Mirabilis jalapa* plant also known as the Marvel of Peru, which alleviates cutaneous discomfort, fades redness of sensitive and reactive skin and strengthens and hydrates the epidermis.

**2) Gel from, for example a firming gel for the body**

| **Ingredient (INCI name)** | **Weight %** |
|---|---|
| **Phase A** | |
| Demineralised water | Qsp 100 |
| Ultrez 10 (Carbomer) | 0.20 |

| **Phase B** | |
|---|---|
| PEG 400 | 5.00 |
| Preservatives | qs |

| **Phase C** | |
|---|---|
| Dimethicone | 4.00 |
| Pemulen TR2 (Acrylates/C10-30 Alkyl Acrylate Cross Polymer) | 0.20 |

| **Phase D** | |
|---|---|
| Tween 20 (Polysorbate 20) | 1.00 |
| **Peptide in a fatty excipient** | 2.00 |

| **Phase E** | |
|---|---|
| Potassium Sorbate | 0.10 |

| **Phase F** | |
|---|---|
| Sodium Hydroxide 30% | 0.60 |
| Demineralised water | 5.00 |

| **Phase G** | |
|---|---|
| Fragrance | 0.10 |

Protocol: Disperse Ultrez 10 in water and let swell for 15 minutes. Heat phase B until dissolved and add to phase A. Weigh and mix phase C. Stir Phase D and add to phase C; mix well. Add phase (C+D) to phase (A+B). Then add phase E. Let swell for 1 hour. Mix well. Neutralize with phase F. Finally, add phase G. pH 6.10.

### Examples of ingredients which may be added to this formulation:

- AQUALANCE™: osmo-protector moisturising active ingredient marketed by Sederma (WO2009/104118) comprising homarine and erythritol.
- LEGANCE™: anti-aging active marketed by Sederma (WO2013/105047), corresponding to a *Zingiber zerumbet Smith* extract obtained by CO₂ supercritical in a water-soluble excipient and titrated in zerumbone ingredient. It is a global anti-aging ingredient for legs. It improves their appearance and comfort by reducing water retention, improving microcirculation and refining adipose tissue.
- BODYFIT™: slimming/firming active ingredient comprising glaucine marketed by Sederma (WO 2004/024695). BODYFIT™ reduces the appearance of cellulite and helps to improve drainage and water distribution in the tissues.
- JUVINITY™: active marketed by SEDERMA reducing signs of aging on the face and neckline, smoothing wrinkles, densifying and restructuring the dermis.

**3) Compact powder form**

| **Ingredient (INCI name)** | **Weight %** |
|---|---|
| **Phase A** | |
| Talc | Qsp 100 |
| Kaolin | 2.00 |
| Calcium Stearate | 1.00 |
| Mica | 4.00 |
| Silica | 1.00 |
| Bismuth Oxychloride | 2.00 |
| Potassium Sorbate | qs |
| Phenoxyethanol | qs |

| **Phase B** | |
|---|---|
| Unipure Black LC 989 HLC [CI 77499 (and) Hydrogenated Lecithin] | 0.20 |
| Unipure Red LC 381 HLC [CI 77491 (and) Hydrogenated Lecithin] | 0.60 |
| Unipure Yellow LC 182 HLC [CI 77492 (and) Hydrogenated Lecithin] | 1.00 |
| Covapearl Star Gold 2302 AS [CI 77891 (and) CI 77491 (and) Synthetic Fluorphlogopite (and) Triethoxycaprylylsilane] | 0.50 |
| Covapearl Brown 838 HLC [CI 77491 (and) Mica (and) Hydrogenated Lecithin) | 1.00 |
| Covapearl Dark Blue 637 [CI 77510 (&) CI 77891 (&) Mica] | 0.10 |

| **Phase C** | |
|---|---|
| Crodamol PTIS-LQ-(MV) [Pentaerythrityl Tetraisostearate] | 4.00 |
| **Peptide according to the invention in a fatty matrix** | 3.00 |

| **Phase D** | |
|---|---|
| Fragrance | 0.30 |

Protocol: Weigh phase A and mix. Weigh phase B and pour into phase B. Pour A+B into the blender and mix. Add phase C to A+B in several times and mix each time. Add phase D. Check homogeneity at every step.

### Example of ingredients which may be added to this formulation:

- VEGESOME MOIST 24™: ingredient marketed by SEDERMA designed for the formulation of moisturizing powder makeup ; it is a powder consisting of hollow particles 25 microns (*Lycopodium clavatum* exins) loaded with an *Imperata cylindrica* extract having moisturizing properties.

**4) Alternative cream form (face or body)**

| **Ingredient (INCI name)** | **Weight %** |
|---|---|
| **Phase A** | |
| Arlacel 170 (Glyceryl Stearate (and) PEG-100 Stearate) | 5.50 |
| Abil Wax 2434 (Stearoxy Dimethicone) | 3.00 |
| Acetulan (Cetyl Acetate (and) Acetylated Lanolin Alcohol) | 1.50 |
| Crodacol C 90 (Cetyl Alcohol) | 1.50 |
| Mineral Oil | 3.00 |
| Shea Butter | 5.00 |
| Unsaponifiable Shea | 1.00 |
| Parsol MCX (Ethylhexyl Methoxicinnamate) | 3.50 |

| **Phase B** | |
|---|---|
| Demineralised water | Qs 100 |

| **Phase C** | |
|---|---|
| Carbopol 940 (Carbomer) | 0.20 |

| **Phase D** | |
|---|---|
| Deminarilised water | 2.00 |
| Triethanolamine 99% | 0.20 |

| **Phase E** | |
|---|---|
| Propylene Glycol | 0.10 |
| Mixed Parabens | |

| **Phase F** | |
|---|---|
| Sodium hydroxyde 30% | 5.00 |
| Demineralised water | qs |

| **Phase G** | |
|---|---|
| **Peptide according to the invention in an hydrophylic matrix** | 2.00 |

Protocol: Weigh phase A and heat at 75°C using a water bath. Weigh phase B and let swell for 20 minutes. Melt phase C until dissolved and add to phase B. Heat phase (B+C) at 75°C using a water bath. Pour phase A into phase (B+C) under Staro stirring. Extemporaneously, add phase D to phase (A+B+C). Approximately at 45°C add phase E and neutralize with phase F. Mix well. At 35°C, add phase G. Homogenize well. pH: 6.20.

### Examples of ingredients which may be added to this formulation:

- SUBLISKIN™: active ingredient marketed by SEDERMA (WO2010/067327) that moisturizes and smooths the skin while allowing it to resist to external aggressions.
- VENUCEANE™: active marketed by Sederma (WO2002/066668) comprising a *Thermus thermophiles* biotechnological extract, that prevents visible signs of photo-aging (spots, wrinkles, dryness ...), protects cell structures from damages caused by UV and strengthens skin integrity.
- KOMBUCHKA™: active ingredient acting on complexion marketed by SEDERMA (WO2004/012650).
- INTENSLIM™: slimming active ingredient marketed by Sederma (WO2013/105048) corresponding to a synergistic combination of extracts obtained by *Globularia cordifolia* plant cell culture, *Zingiber zerumbet Smith* titrated in zerumbone and vegetable caffeine obtained by supercritical CO₂ extraction.

## Claims

1. Tripeptide having X-KGH-Z or X-KHG-Z formula; wherein:
• At the N-terminal end X is selected from H, -CO-R₁ and -SO₂-R₁;
• At the C-terminal end Z is selected from OH, OR₁, NH₂, NHR₁ or NR₁R₂; and
• R₁ and R₂ are, independently from each other, an alkyle chain of 1 to 24 carbon atoms, except tripeptides wherein (X=H and Z=OH) or (X=H and Z=NH₂) and except the tripeptide KHG-N-octyl ester wherein X=H and Z=OR₁ with R₁ being an alkyl chain of 8 carbon atoms.

2. Tripeptide according to claim 1, wherein R₁ and R₂ are, independently from each other, an alkyle chain of 3 to 24 carbon atoms.

3. Tripeptide according to claim 1 or 2, **characterized in that** X is an acyle radical -CO-R₁ and at the C-terminal end Z is a radical selected from OH, OMe, OEt and NH₂.

4. Tripeptide according to one of the preceding claims, **characterized in that** the acyle radical - CO-R₁ is selected from an octanoyl (C₈), decanoyl (C₁₀), lauroyl (C₁₂), myristoyl (C₁₄), palmitoyl (C₁₆), stearoyl (C₁₈), biotinoyl, elaidoyl, oleoyl and lipoyl.

5. Tripeptide according to claim 4, **characterized in that** Z is OH.

6. Tripeptide according to claim 5, corresponding to Pal-KGH-OH or Pal-KHG-OH.

7. Cosmetic composition comprising as active ingredient an effective amount of at least one tipeptide according to anyone of the preceding claims in a physiologically acceptable medium.

8. Composition according to claim 7, **characterized in that** the effective amount is comprised between 0.000001% and 15% with regard to the total weight of the composition.

9. Composition according to claim 8, **characterized in that** the effective amount is comprised between 0.0001% and 5% with regard to the total weight of the composition.

10. Composition according to anyone of claims 7 to 9, **characterized in that** it comprises at least one additional active ingredient selected from vitamin B3 compounds, niacinamide, tocopherol, retinoid compounds, hexamidine, α-lipoic acid, resveratrol, DHEA, hyaluronic acid and peptides.

11. Composition according to anyone of claims 7 to 10, in the form of solution, dispersion, emulsion, paste, or powder, individually or as a premix or in vehicles individually or as a premix in vectors such as macro-, micro-, or nano-capsules, macro-, micro- or , nano-spheres, liposomes, oleosomes or chylomicrons, macro-, micro-, or nanoparticles or macro- micro- or nano-sponges, micro- or nano-emulsions or adsorbed on organic polymer powders, talcs, bentonites, spores or exines, and other inorganic or organic supports.

12. Use of at least one tripeptide according to anyone of claims 1 to 6 or a composition according to anyone of claims 7 to 11 for a non therapeutical cosmetic treatment to improve the general state of the skin and its appendages and to treat their imperfections.

13. Use according to claim 12, for a topical treatment.

14. Use according to claim 12 or 13, for an anti-ageing treatment.

15. Use according to anyone of claims 12 to 14 for:
- A treatment of wrinkles and fine lines; and/or
- Improving the mechanical properties of the skin: firmness, tone, elasticity and/or flexibility; and/or
- Increasing density and volume of skin (volumizing, plumping and/or restructuring effect); and/or
- To fight strechmarks, and/or
- To improve homogenity and radiance of complexion.

## Patentansprüche

1. Tripeptid mit der Formel X-KGH-Z oder X-KHG-Z; wobei:
• am N-terminalen Ende X aus H, -CO-R₁ und -SO₂-R₁ ausgewählt ist;
• am C-terminalen Ende Z aus OH, OR₁, NH₂, NHR₁ oder NR₁R₂ ausgewählt ist; und
• R₁ und R₂ unabhängig voneinander eine Alkylkette mit 1 bis 24 Kohlenstoffatomen sind, mit Ausnahme von Tripeptiden, bei denen (X = H und Z = OH) oder (X = H und Z = NH₂), und mit Ausnahme des Tripeptids KHG-N-Octylester, wobei X = H und Z = OR₁, wobei R₁ eine Alkylkette mit 8 Kohlenstoffatomen ist.

2. Tripeptid nach Anspruch 1, wobei R₁ und R₂ unabhängig voneinander eine Alkylkette mit 3 bis 24 Kohlenstoffatomen sind.

3. Tripeptid nach Anspruch 1 oder 2, **dadurch gekennzeichnet, dass** X ein Acylrest -CO-R₁ ist, und am C-terminalen Ende Z ein Rest ausgewählt aus OH, OMe, OEt und NH₂ ist.

4. Tripeptid nach einem der vorhergehenden Ansprüche, **dadurch gekennzeichnet, dass** der Acylrest -CO-R₁ ausgewählt ist aus Octanoyl (C₈), Decanoyl (C₁₀), Lauroyl (C₁₂), Myristoyl (C₁₄), Palmitoyl (C₁₆), Stearoyl (C₁₈), Biotinoyl, Elaidoyl, Oleoyl und Lipoyl.

5. Tripeptid nach Anspruch 4, **dadurch gekennzeichnet, dass** Z OH ist.

6. Tripeptid nach Anspruch 5, das Pal-KGH-OH oder Pal-KHG-OH entspricht.

7. Kosmetische Zusammensetzung, umfassend als Wirkstoff eine wirksame Menge mindestens eines Tripeptids nach einem der vorhergehenden Ansprüche in einem physiologisch verträglichen Medium.

8. Zusammensetzung nach Anspruch 7, **dadurch gekennzeichnet, dass** die wirksame Menge zwischen 0,000001 % bis 15 %, bezogen auf das Gesamtgewicht der Zusammensetzung, liegt.

9. Zusammensetzung nach Anspruch 8, **dadurch gekennzeichnet, dass** die wirksame Menge zwischen 0,0001% bis 5%, bezogen auf das Gesamtgewicht der Zusammensetzung, liegt.

10. Zusammensetzung nach einem der Ansprüche 7 bis 9, **dadurch gekennzeichnet, dass** sie mindestens einen zusätzlichen Wirkstoff umfasst, der aus Vitamin-B3-Verbindungen, Niacinamid, Tocopherol, Retinoidverbindungen, Hexamidin, α-Liponsäure, Resveratrol, DHEA, Hyaluronsäure und Peptiden ausgewählt ist.

11. Zusammensetzung nach einem der Ansprüche 7 bis 10 in Form einer Lösung, Dispersion, Emulsion, Paste oder eines Pulvers, einzeln oder als Vormischung oder in Vehikeln einzeln oder als Vormischung in Vektoren wie Makro-, Mikro- oder Nano-Kapseln, Makro-, Mikro- oder Nanokugeln, Liposomen, Oleosomen oder Chylomikronen, Makro-, Mikro- oder Nanopartikeln oder Makro-, Mikro- oder Nanoschwämmen, Mikro- oder Nanoemulsionen oder adsorbiert auf organischen Polymerpulvern, Talkum, Bentonit, Sporen oder Exinen und anderen anorganischen oder organischen Trägern.

12. Verwendung mindestens eines Tripeptids nach einem der Ansprüche 1 bis 6 oder einer Zusammensetzung nach einem der Ansprüche 7 bis 11 für eine nichttherapeutische kosmetische Behandlung zur Verbesserung des allgemeinen Zustands der Haut und ihrer Anhänge und zur Behandlung ihrer Unvollkommenheiten.

13. Verwendung nach Anspruch 12 für eine topische Behandlung.

14. Verwendung nach Anspruch 12 oder 13 für eine Anti-Aging-Behandlung.

15. Verwendung nach einem der Ansprüche 12 bis 14 zur:
- Behandlung von Falten und feinen Linien; und/oder
- Verbesserung der mechanischen Eigenschaften der Haut: Festigkeit, Spannkraft, Elastizität und/oder Flexibilität; und/oder
- Erhöhung der Hautdichte und des Hautvolumens (voluminisierende, prallmachende und/oder restrukturierende Wirkung); und/oder
- Bekämpfung von Dehnungsstreifen und/oder
- Verbesserung der Homogenität und Ausstrahlung des Teints.

## Revendications

1. Tripeptide de formule X-KGH-Z ou X-KHG-Z ; dans lequel :
• en extrémité N-terminale, X est choisi parmi H, -CO-R₁ et -SO₂-R₁;
• en extrémité C-terminale, Z est choisi parmi OH, OR₁, NH₂, NHR₁ et NR₁R₂ ; et
• R₁ et R₂ sont, indépendamment l'un de l'autre, une chaîne alkyle de 1 à 24 atomes de carbone,
à l'exception des tripeptides dans lesquels (X=H et Z=OH) ou (X=H et Z=NH₂) et
à l'exception du tripeptide KHG-N-ester d'octyle dans lequel X=H et Z=OR₁ avec R₁ étant une chaîne alkyle de 8 atomes de carbone.

2. Tripeptide selon la revendication 1, dans lequel R₁ et R₂ sont, indépendamment l'un de l'autre, une chaîne alkyle de 3 à 24 atomes de carbone.

3. Tripeptide selon la revendication 1 ou 2, **caractérisé en ce que** X est un groupement acyle -COR₁ et à l'extrémité C-terminale Z est un groupement choisi parmi OH, OMe, OEt et NH₂.

4. Tripeptide selon l'une des revendications précédentes, **caractérisé en ce que** le groupement acyle -CO-R₁ est choisi parmi un octanoyle (C₈), decanoyle (C₁₀), lauroyl (C₁₂), myristoyle (C₁₄), palmitoyle (C₁₆), stéaroyle (C₁₈), biotinoyle, élaïdoyle, oléoyle et lipoyle.

5. Tripeptide selon la revendication 4, **caractérisé en ce que** Z est OH.

6. Tripeptide selon la revendication 5, correspondant au Pal-KGH-OH ou au Pal-KHG-OH.

7. Composition cosmétique comprenant comme ingrédient actif une quantité efficace d'au moins un tripeptide selon l'une quelconque des revendication précédentes dans un milieu physiologiquement acceptable.

8. Composition selon la revendication 7, **caractérisée en ce que** la quantité efficace est comprise entre 0.000001% et 15% par rapport au poids total de la composition.

9. Composition selon la revendication 8, **caractérisée en ce que** la quantité efficace est comprise entre 0.0001% et 5% par rapport au poids total de la composition.

10. Composition selon l'une quelconque des revendications 7 à 9, **caractérisée en ce qu'**elle comprend au moins un ingrédient actif additionnel choisi parmi les composés de la vitamine B3, la niacinamide, le tocophérol, les composés rétinoïdes, l'hexamidine, l'acide α-lipoïque, le resvératrol, la DHEA, l'acide hyaluronique et les peptides.

11. Composition selon l'une quelconque des revendications 7 à 10, sous la forme d'une solution, d'une dispersion, d'une émulsion, d'une pâte ou d'une poudre, seule ou en tant que pré-mélange ou dans des véhicules individuellement ou en tant que pré-mélange dans des vecteurs comme les macro-, micro-, ou nano-capsules, macro-, micro- ou , nano-sphères, les liposomes, les oléosomes ou les chylomicrons, macro-, micro-, ou nanoparticules ou macro- micro- ou nanoéponges, micro- ou nano-émulsions ou adsorbée sur des poudres polymères organiques, des talcs, des bentonites, des spores ou exines et d'autres supports organiques ou minéraux.

12. Utilisation d'au moins un tripeptide selon l'une quelconque des revendications 1 à 6 ou d'une composition selon l'une quelconque des revendications 7 à 11 pour un traitement cosmétique non-thérapeutique pour améliorer l'état général de la peau et de ses phanères et en traiter ses imperfections.

13. Utilisation selon la revendication 12, pour un traitement topique.

14. Utilisation selon la revendication 12 ou 13, pour un traitement anti-vieillissement.

15. Utilisation selon l'une quelconque des revendications 12 à 14 pour :
- un traitement des rides et ridules ; et/ou
- améliorer les propriétés mécaniques de la peau : fermeté, tonicité, élasticité et/ou souplesse ; et/ou
- augmenter la densité et le volume de la peau (effet volumateur, repulpant et/ou restructurant) ; et/ou
- lutter contre les vergétures, et/ou
- améliorer l'homogénéité et l'éclat du teint.
